# EUROPEAN PATENT APPLICATION

(11) **EP 1 057 481 A1**
(43) Date of publication of application: **06.12.2000**
(21) Application number: 99972098.0
(22) Date of filing: 12.10.1999
(51) Int. Cl.: A61K 9/70, A61K 47/38

(54) **AGENTS FOR REPAIRING DAMAGED TISSUE SITES**

(30) Priority: 18.11.1998 JP 32785598
(71) Applicant: Medical Industries Corporation, Tokyo 113-0034 (JP)
(72) Inventor: SUZUKI, Shigeki, Tokyo 154-0002 (JP)
(74) Representative: Weisert, Annekäte, Dipl.-Ing. Dr.-Ing.
(86) International application number: JP9905603
(87) International publication number: WO0028978

(57) **Abstract**

The present invention relates to a new agent for repairing the damaged tissue and promoting a healing effect on external injuries or wounds, comprising of carboxymethyl cellulose, in which drug applicable to a wounded surface is combined with the said fibrous carboxymethyl cellulose by ionic bonding, affinity bonding or mechanical bonding, and the fibrous carboxymethyl cellulose is obtained from natural or regenerated cellulose by chemical treatment and by carboxylmethylation.

## Description

### TECHNICAL FIELD

The present invention relates to an agent for repairing damaged tissues. More specifically, it relates to an agent which promotes the healing of the damaged tissues from surgical injuries or wounds, in which a drug is dispersed in or combined with absorbable hemostatic cellulose obtained by the chemical process of fibrous carboxymethyl cellulose, especially natural or regenerated cellulose fibers.

### BACKGROUND ART

Since before, a large number of hemostatic materials have been available for use on operations, such as external injuries, bleeding inside cavities during surgical operations, burns, in obstetric and gynecological treatments, extractions in oral cavities or dentistry, and hemorrhoidectomy. The examples include hemostatic powders used for simple external injuries in daily life, hemostatic gelatin sponges (collagen sponges) used for absorbing the body fluids, or hemostatic gauzes.

These hemostatic materials are directly applied to the surface of the bleeding wound; however, they have not shown sufficient effectiveness yet. For example, the hemostatic powder requires additional other hemostatic agents, and it has a problem that its quality changes due to disinfect by high temperature/pressure. In addition, due to the low absorbability of the powders, they are difficult to be absorbed in the body, consequently, easily causes foreign body reaction. On the other hand, the collagen sponge, gelatin sponge, is traditional surgical hemostatic material, which is absorbed in the body. However, the ingredient is protein, which easily causes putrescence by the conditions of the applied region. As a result, it may cause infections. In addition, gelatin sponges easily come free from the wounded surface due to their low viscosity, and have effects on the ability to impregnate the wound with blood clots.

On the other hand, oxycellulose (oxidized cellulose) hemostatic gauze, oxidized after becoming compounded ingredients in which cellulose derivatives are impregnated on a cloth of cellulose, is widely used as an absorbable hemostatic material. However, this material is difficult to be solved, and takes considerable long time to be absorbed into the body. Furthermore, its relative high cost prevents it from everyday use; therefore, it is demanded to develop the widely available novel hemostatic material, which accomplishes precise hemostatic controls of bleeding in the body, has quick hemostatic effects, and is absorbable on the wounded surface without any foreign body reaction.

The present inventor has proposed, this new type of absorbable hemostatic cellulose as a hemostatic material having the above-mentioned demands after he confirmed the followings. The carboxymethyl cellulose fiber, obtained from the natural or regenerated cellulose by chemical processes, quickly dissolves and expands when it contacts with the body fluids such as blood. After that, it goes into a colloidal state, covers the wounded surface to stop bleeding, and accomplishes hemostatic effects (Japanese Laid-Open Patent Publication No. Hei 10-77571).

### DISCLOSURE OF THE INVENTION

The purpose of the present invention is to provide a new agent for promoting the healing of damaged tissue, in which the aforementioned absorbable cellulose is applied in order to promote healing effects on damaged tissues, such as external wounds, more effectively.

One purpose of the present invention to solve the above-mentioned problems is to provide an agent for repairing the damaged tissues. In the agent, fibrous carboxymethyl cellulose is combined with drug applicable to the wounded surface by ionic bonding, affinity bonding or mechanical bonding.

As the basic embodiment, the following cellulose is used as the fibrous carboxymethyl cellulose in the present invention. First, natural or regenerated cellulose fiber is processed with an alcoholic aqueous solution of alkali metal hydroxide, and reacted with monohalogenated acetic acid. Next, the hydroxyl group, in the anhydrous glucose unit consisting of the cellulose molecules, is changed into a carboxymethyl group. Then the cellulose is obtained after the cellulose fiber is purified.

As another embodiment, this invention uses the following cellulose. First, the above-said natural or regenerated cellulose to be used is previously treated with an aqueous solution of sodium hypochlorite and hydrogen peroxide for water-solubilizing process. Second, it is processed with the alcoholic aqueous solution of alkali metal hydroxide, and reacted with monohalogenated acetic acid to introduce the carboxymethyl group into the hydroxyl group in the anhydrous glucose unit of the cellulose molecules. Finally, it is purified; as a result, the cellulose to be used is obtained.

By these chemical processes, the natural or regenerated cellulose fiber is water-solubilized, especially resulting from the carboxymethylation of all or a portion of the primary hydroxyl groups in the anhydrous glucose unit of the cellulose molecules. In the present invention, such water-soluble cellulose thus obtained is preferably used

Accordingly, as the second purpose of the present invention is to provide the special water-soluble hemostatic cellulose, mentioned above, in the form of fiber or gauze, which can be used in the agent for repairing the damaged tissues.

### BRIEF DESCRIPTION OF DRAWINGS

FIG. 1 shows the profile of the agglutination of fibrin monomer, which is one of hemostatic effect of the present absorbable cellulose.
FIG. 2 shows the mechanism of the blood coagulation system to explain the hemostatic effect of the present absorbable cellulose.

### BEST MODE FOR CARRYING OUT THE INVENTION

The absorbable hemostatic cellulose, fibrous carboxymethyl cellulose, to be used in the agent for repairing the damaged tissue of the present invention, is obtained through processing chemically the natural and regenerated cellulose fibers. This absorbable hemostatic cellulose excels in absorption of tissue fluids such as blood, and expands approximately five times as much as the original volume after the absorption. Especially, when it contacts with blood, it dissolves rapidly, and covers the wound going into a colloid state with blood. As a result, it raises the hemostatic effect.

In addition, the carboxymethyl cellulose obtained by the chemical process, which is used in this invention, is chemically stable without toxicity. Moreover, it is absorbed easily into the body, so that it has a more superior effect to those of other conventional ones.

Therefore, the absorbable hemostatic cellulose of the invention can be used as an extremely effective hemostatic material in itself.

One example of these absorbable hemostatic celluloses is obtained as follows. First, the natural or regenerated cellulose is processed with an alcoholic aqueous solution of alkali metal hydroxide, and reacted with monohalogenated acetic acid. Consequently, the hydroxyl group in the anhydrous glucose unit of the cellulose molecules is introduced into the carboxymethyl group, and then the cellulose is purified.

Furthermore, another example of these absorbable hemostatic celluloses is also obtained as follows. For the above-mentioned carboxymethyl introduction, the natural or regenerated cellulose is previously treated with an aqueous solution of sodium hypochlorite and hydrogen peroxide. Additionally, it is processed with an alcoholic aqueous solution of alkali metal hydroxide, the same one as the above, and is reacted with monohalogenated acetic acid to introduce the carboxymethyl group into the hydroxyl group in the anhydrous glucose unit of the cellulose molecules. Finally, it is purified.

All or a portion of the primary hydroxyl groups, presented in the anhydrous glucose unit, are carboxymethylated in the absorbable cellulose molecules thus obtained. That is, there are three (3) hydroxyl groups in the anhydrous glucose units of the cellulose molecules, and logically the degree of etherification becomes three (3) due to these carboxymethyl groups. However, the degree of etherification in the absorbable cellulose of the invention is especially more than 1.0.

Due to the degree of that etherification, the absorbable cellulose of the present invention exceeds in absorption of tissue fluids such as blood, and expands in the volume following the absorption. Especially, when it contacts with blood, it dissolves quickly, and makes a gelatin form with blood, covering the wounded surface. As a result, it increases hemostatic effect.

The said absorbable hemostatic cellulose has the structural units represented by the following formula (I) in the molecules.

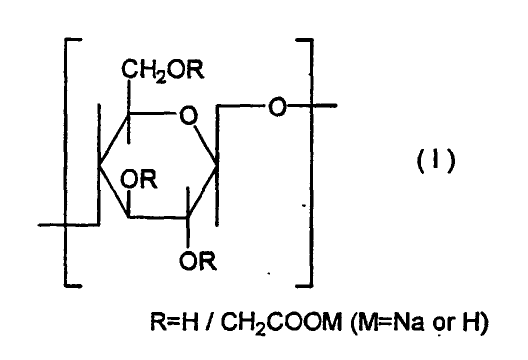

That is, the hydroxyl groups in the anhydrous glucose unit of the said cellulose molecules are either totally or partially carboxymethylated. In addition, the carboxyl group of the carboxymethyl group forms its sodium salt, or forms a chemical structural unit in which the hydroxyl groups exist as a mixture of free carboxylic acids.

However, the absorbable hemostatic cellulose to be used in this invention is not limited only to the one having the structural unit represented by the above-mentioned formula (I). Of course, the any other cellulose, mentioned as follows, is included. In the cellulose, hydroxyl group from within the anhydrous glucose unit is partially carboxymethylated (etherificated) by the aforementioned chemical process of the natural or regenerated cellulose. As a result, the cellulose displays the desired absorbable hemostatic effect.

The drugs, combining with the absorbable hemostatic cellulose and applicable to the wounded surface as the agent for promoting the healing of the damaged tissues in the present invention, may include antibacterial agents, antifungal agents, steroids, anti-inflammatory agents, antihistamines, cell growth factors, growth factors anti-agents and the like.

These drugs may be combined with the cellulose alone or together.

In the agent for repairing the damaged tissues of the present invention, the drug applicable to the wounded surface may be bonded with the said absorbable hemostatic cellulose by ionic, affinity, or mechanical bond.

For example, in the case of the mechanical bond, the powder of the suitable drug is dry-kneaded together with the powdery soluble hemostatic cellulose to form the agent for promoting the healing of the damaged tissues of the present invention.

Furthermore, not only the powdered drugs but also the absorbable hemostatic cellulose may be used for the above purpose after the same procedures.

On the other hand, the ionic bond or affinity bond is accomplished by bonding with suitable drugs during the manufacturing process of the absorbable cellulose used in the present invention. For example, in this aqueous ethanol process in the Manufacturing Example as described later, the suitable drug is dissolved in the aqueous ethanol solution and is adjusted about the pH of the solution. Consequently, the absorbable hemostatic cellulose is manufactured in which the drug is ionic or affinity bonded with the cellulose. After that, it is obtained as the agent for the promotion of the healing of damaged tissues of the present invention.

The agent for promoting the healing damaged tissues of the present invention thus obtained absorbs body fluids such as blood, and expands when applied to the wounded region. According to the expansion of the cellulose, the drug combining with the said cellulose is released to heal the damaged tissue effectively.

It became clear that the releasing speed of the drug from the cellulose is controlled by the numbers of carboxymethyl groups at the hydroxyl group presented in the anhydrous glucose units of the cellulose molecules.

Therefore, by adjusting the degree of etherification of the absorbable hemostatic cellulose, the desirable releasing speed of the drugs is controlled. Particularly, the cellulose, having the degree of etherification being more than 1.0, is preferably used in the present invention.

### Example

The unique action of the absorbable hemostatic cellulose to be used in the present invention is described as follows. Furthermore, concerning the concrete hemostatic test and the agent for promoting the healing of damaged tissues of the present invention, the concrete performed examples are explained.

One example of the preferable hemostatic cellulose used in the present invention may be the absorbable hemostatic cellulose obtained as follows. First, the natural or regenerated cellulose (for example, cellulose fiber) is processed by the specified chemical treatment. In other words, it is impregnated in the aqueous alcoholic solution of alkali metal hydroxide. Then, it is reacted with monohalogenated acetic acid to introduce carboxylmethyl groups at the hydroxyl groups of the anhydrous glucose unit of the cellulose molecules, and purified.

The alkali metal hydroxide to be used in this process includes sodium hydroxide and potassium hydroxide and the like, and among them, sodium hydroxide is preferably used.

The alcoholic aqueous solution for the sodium hydroxide may be methanol, ethanol, isopropyl alcohol and the like, and among them, ethanol is preferably used. For the ethanol aqueous solution in the sodium hydroxide, it is suitable that the mixture solution of from about 40% to 50%, preferably about 45% aqueous sodium hydroxide solution and from 90% to 98%, preferably about 95% ethanol solution is reacted at room temperature.

Then, the natural or regenerated cellulose thus treated is reacted with the monohalogenated acetic acid, preferably with a reactive solution of the monohalogenated acetic acid, to introduce methylcarboxyl groups at the hydroxyl groups of the anhydrous glucose units of the cellulose molecules. As the reactive solution of the monohalogenated acetic acid, 95% ethanol solution to the monochloroacetic acid is preferably used. This reaction may be accomplished by mixing the cellulose fibers obtained by the aforementioned process with the reactive solution of mono-chloroacetic acid, at room temperature, for about 6 to 10 hours.

Then, the pH of the cellulose fibers is adjusted to 6.5 to 7.5 by the mixture solution of 20% hydrochloric acid and about 70% to 80%, preferably 75% ethanol. Moreover, the fibers is washed with about 75% ethanol until the amount of sodium chloride in the fibers becomes less than 1% by the weight, dried and sterilized to obtain the absorbable hemostatic cellulose to be used in the present invention.

The absorbable hemostatic cellulose thus obtained has the chemical structural units represented in the formula (I).

In addition, following the completion of reacting with monochloroacetic acid in the manufacturing process described above, the suitable drug, which can be applied to a wounded surface, is dissolved in 70% to 85%, preferably 75% ethanol solution, and the pH is adjusted. As a result, the absorbable hemostatic cellulose combined with the drug through ionic or affinity bonding can be produced.

Furthermore, another preferable hemostatic cellulose used in the present invention may be obtained in the following manner. Namely, the natural or regenerated cellulose fibers are treated. with an aqueous solution of sodium hypochlorite and then, with an aqueous solution of hydrogen peroxide respectively. The condition of this process is not limited to particularly; however, the treatment with the aqueous solution of sodium hypochlorite may be conducted under alkali conditions, preferably with a pH level of 9 to 10 by churning for 40 to 60 minutes. Then, the treatment with the aqueous solution of hydrogen peroxide may be conducted under the same kind of alkali condition, by using about 3% of hydrogen peroxide aqueous solution, in the presence of a small amount of sodium pyrophosphate, for 50 to 70 minutes.

Then, the cellulose fibers thus treated are further processed in the same manner as described above to produce another absorbable hemostatic cellulose to be used in the present invention.

The soluble hemostatic cellulose obtained in this process also has the same chemical structural unit represented by the formula (I).

Furthermore, as the same as described above, especially, following the completion of reacting with monochloroacetic acid, the suitable drug, which can be applied to a wounded surface, is dissolved in 70% to 85%, preferably 75% ethanol solution dissolving, and the pH is adjusted. In this way, the absorbable hemostatic cellulose, by ionic bonding or affinity bonding with the drug, can also be produced.

The absorbable hemostatic cellulose, to be used in the agent for the promotion of the healing of damaged tissue in this invention produced by the above manner, increases the concentration and viscosity of the blood and tissue fluid while contacting with the wounded surface. Then, in order to reduce the speed of flow of the blood and tissue fluid, for example, the cellulose absorbs much of the water in the bloodstream, expands more than five times as much as its original volume, and becomes colloidal, form after absorption of the water. Therefore, the wound surface is covered, and the ends of the blood capillaries are blocked to show the excellent hemostasis effects.

By the way, the following has been known about the activated cascades of enzymes in the blood coagulation system. First, it converts prothrombin to thrombin in the final stage, and this thrombin converts fibrinogen to fibrin monomer, and this results in that fibrin monomer aggregates to form fibrin polymer. Furthermore, it has been known that through activated XIII factors, hemostasis is completed by the formation of cross-linking fibrin polymer.

Based on these points, the effects of the soluble hemostatic cellulose for the prothrombin formation time (PT), the activated partial thromboplastin formation time (APTT), as well as thrombin formation time (TT) were examined in comparison with no using hemostatic cellulose. The absorbable hemostatic cellulose used in the experiment was obtained in Manufacturing Example 1, mentioned later. The results were summarized in Table 1 below.

**Table 1**

| Results of PT (prothrombin time), APTT (activated partial thromboplastin time) and TT (thrombin time) | | |
|---|---|---|
| | No hemostatic agent | Soluble hemostatic cellulose of the present invention |
| PT (sec.) | 22.2 | 20.9 |
| APTT (sec.) | 52.0 | 53.5 |
| TT (sec.) | 12.3 | 11.6 |

As is apparent from the Table 1 above, the absorbable hemostatic cellulose of the present invention showed almost no effect on the activation of enzymes in the blood coagulation system; however, a remarkable hemostatic effect was displayed in animal testing. Therefore, the effectiveness on the agglutination of fibrin monomer was investigated in order to discover the effective mechanism of this unique hemostatic effect of the absorbable hemostatic cellulose of the present invention.

The steps for the agglutination of fibrin are the time required for the formation of two strand chain fibers having the appropriate length, which have been polymerized by the related fibrin monomer (time lag). Another is the step where the chain fibers, having the appropriate length, are bonded and form a three dimensional network.

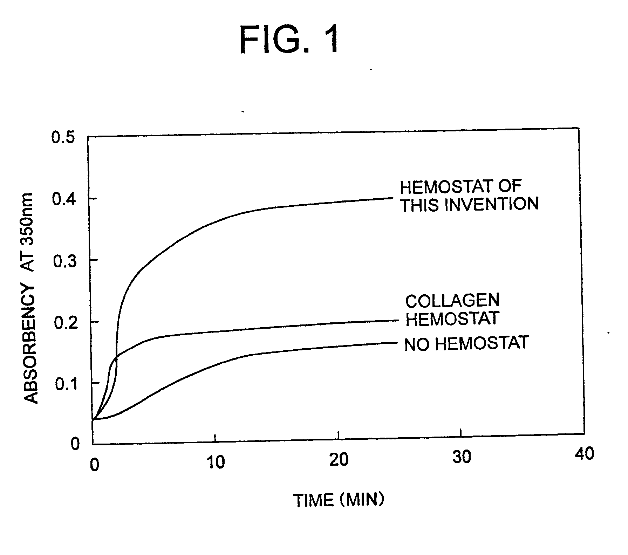

To compare the absorbable hemostatic cellulose of the present invention with hemostatic materials of collagen on the market, in FIG. 1, it is shown that the profile of the agglutination of fibrin monomer is observed at an absorbency of 350 nm. In the FIG. 1, the profile of the agglutination of fibrin monomer using no hemostat is also shown.

As it is apparent from the FIG. 1, in the agglutination of fibrin by these hemostatic materials, the existence of the lag time as well as the increase of absorbency, which reflects the formation of a side-by-side bond of these two strand chain fibers, were observed. Ultimately, it became maximum absorbency, and stable.

Therefore, the absorbable hemostatic cellulose of the present invention reduces the lag time and promotes the increase of maximum absorbency in the same way as the other hemostatic materials of collagen on the market. That is, the both hemostatic materials have absolutely no effect on the activation of coagulation system enzymes, and by promoting the agglutination of fibrin monomer formed in the normal coagulation reaction to appear the hemostatic effects. Particularly, the absorbable hemostatic cellulose of the present invention remarkably promotes the agglutination of fibrin monomer in comparison with the other hemostatic materials of collagen.

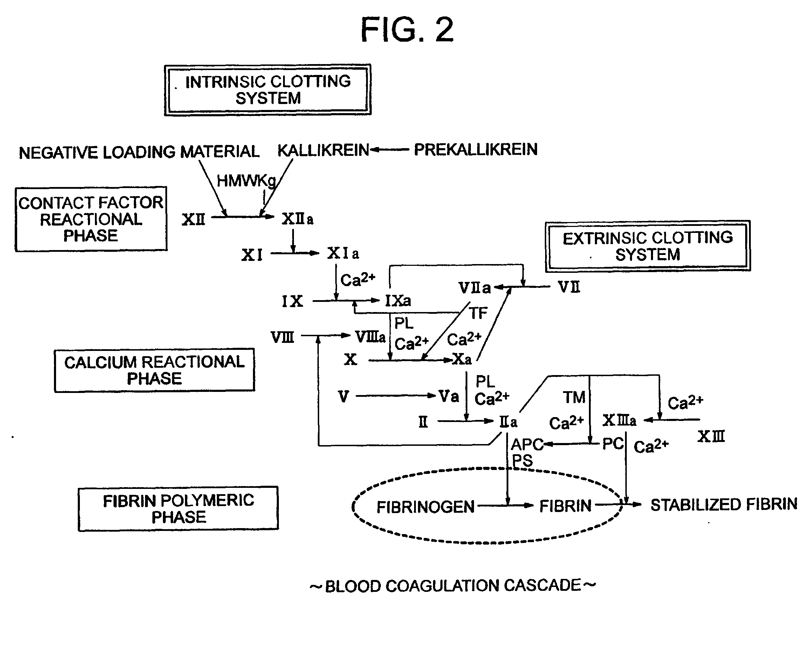

FIG. 2 shows the mechanism of the blood coagulation system. The absorbable hemostatic cellulose of the present invention is promoting the final step shown in the coagulation cascade boxed with the dotted line; furthermore, even in comparison with collagen hemostats generally sold on the market, the promotion of an excellent agglutination was realized.

The following shows the unique hemostatic effects and the internal absorbency effects, as mentioned above, of the absorbable hemostatic cellulose of the present invention, which are observed by the hemostatic effects in bleeding of rats' livers, as well as be histopathological tests on the surgical area.

### Hemostatic Test 1: Hemostatic effect and the test for absorbency inside the body (Part 1)

### [Purpose]

The hemostatic effect of the absorbable cellulose of the invention on a bleeding wound (the excision of a 2 cm square area of the liver tissue) in a body cavity of a rat was examined. Furthermore, the healing process of that wounded area, a residual state of the cellulose in the body, and the local irritation were also examined.

### [Test method]

Seven rats per group, 5-weeks old Wister-strain males, were used. The rats were anesthetized by intraperitoneal administration of pentobarbital (30 mg/kg), and a median line incision was made in the abdomen of each rat, and 10 x 10 cm square of the inferior margin of the livers was cut off using a surgical knife.

As a specimen, 10 x 20 cm square of the woven fabric, of the absorbable hemostatic cellulose obtained in the Manufacturing Example 1, was formed as a test sample. As a control, fine fibrous collagen on the market (trade name: Aviden) was formed in the same way.

As a method of hemostasis, each specimen was applied, like covered, to the wounded area without pressure on the local area. If the specimen was observed to be no longer able to absorb blood from the specimen, new specimens were applied one by one until the bleeding stopped, and the amount of time and the number of specimens required were recorded.

Then, the abdomen was closed immediately upon the observation of the hemostatis, and reopened thirty days later to determine the healing effect of the wounded area as well as the residual state of the specimens by macroscopic method. Subsequently, the liver was extirpated and fixed with 10% formalin solution, and the specimen of the liver was made and thistopathologically examined.

### [Results]

The time for the hemostasis and the amount of the specimens needed are summarized in Table 2.

**Table 2**

| Test group | Average time required for hemostasis (seconds) | Samples required for hemostasis | |
|---|---|---|---|
| | | Average number of sample | Cavity ratio |
| Test sample | 190.14 (n=7) | 2 | 1.00 |
| Control | 198.43 (n=7) | 3 | 3.38 |

With respect to the operability, the absorbable hemostatic cellulose of the present invention was easier to handle with surgical instruments than the control sample, besides it maintained the original shape until the application. Furthermore, it was well adhered on the surgical area immediately upon being applied, and showed excellent hemostatic effect.

On the other hand, the control sample was fragile, so that when it was handled by surgical instruments or when the desired area of application was not a flat surface, it tended to fold or break by bending, resulting in effect on its operability.

By the autopsy and pathologic examination on the thirtieth day after the operation, no implant of the cellulose of the present invention was observed by the optical microscopic check, and it was determined that the cellulose had been completely absorbed.

In addition, the majority of the excised area of the liver showed signs of complete healing, and some portions having skin hypertrophy were observed. Moreover, some areas remaining small degree of repairing by granulation tissue were also observed; however, the liver parenchyma had no remarkable changes.

On the other hand, the materials of the specimens still remained in the excised portions of the liver, and the materials had been hardly absorbed. Also, the middle level of foreign body inflammatory reactions was observed in the operating area of the specimens as well as in the surrounding tissue.

As is clearly ascertained from the these results, the soluble hemostatic cellulose of the present invention showed a short period of time for hemostasis on the injured area, and required only a small amount of test material. Therefore, the operability of the cellulose of the present invention is excellent, and the shorter surgical time can be achieved.

### Hemostatic Test 2: The Hemostatic effect and the test for absorbency inside the body (Part 2)

### [Purpose]

From the results of the Hemostatic Test 1 (Part 1) above, due to the fact that the absorbable hemostatic cellulose of the invention did not remain in the surgical area and had been absorbed in the living body on the first month after the operation. For this reason, a test was conducted in the same way making observations in shorter time periods. In short, the hemostatic effect on the bleeding wounds of body cavities of rats (the excision of 2 cm square of liver tissue), the healing status of the wounded area, residual status of the hemostatic cellulose in the body, and local irritation were examined on the first day, the first week, and the second week after the operation.

The test method was the same as Part 1; however, nine rats per group were used in this test. As the test groups, rats were treated with the cellulöse of the present invention. As the control group (1), rats were treated without hemostatic materials and the abdomen was immediately closed, then, the gauze of *Japanese Pharmacopoeia* covered the suture parts for the hemostasis. As the control group (2), rats were treated with the fine fibrous collagen on the market (trade name: Aviden).

### [Results]

The time and the amount of the specimens required for hemostasis are summarized in Table 3.

**Table 3**

| Test group | Average time required for hemostasis (seconds) | Samples required for hemostasis | |
|---|---|---|---|
| | | Average number of sample | Cavity ratio |
| Control (1) | 170.25 (n=8) | 3.38 (n=9) | |
| Test sample | 123.86 (n=9) | 1.89 (n=9) | 1.00 |
| Control (2) | 198.00 (n=9) | 2.33 (n=9) | 3.38 |

As apparent from the Table 3 above, the test group, treated with the soluble hemostatic cellulose of the present invention, achieved hemostasis in a shorter time, and required a smaller amount of the specimens in comparison with the control groups.

From the post-operative autopsy and pathologic examinations, even at the first day after the operation, no implant was observed around the operated liver area in the group treated with the absorbable hemostatic cellulose of this invention under a light microscopic examination. Additionally, the cellulose had been absorbed in the living body.

In the case of control group (2), in which the rats were treated with the fine fibrous collagen on the market, some materials remained even on the first month after the operation, and it was reconfirmed that the material is a difficult to be absorbed. Also, the foreign body inflammatory reactions were already observed in the operating area of the test materials as well as in the surrounding tissue even on the first day after the operation, and the inflammatory reactions still remained on the first week after the operation, and their recovery was slow.

As a contrast, in the control group (1), rats were treated with no hemostatic agent, and these suture parts were covered only by gauze of *Japanese Pharmacopoeia*. In this case, the acute foreign body inflammatory reactions were already observed on the first day after the operation, and the mild level of repairing the inflammation was observed on the first week after the operation.

Judging from the results of these hemostatic tests, it can be concluded that the absorbable hemostatic cellulose of the present invention is exceedingly safe, shows immediate hemostatic effect, and is easy to use. Therefore, it was also concluded that this would be a useful medicinal material for actual use.

There was no change in the hemostatic effectiveness of the absorbable hemostatic cellulose of the present invention, showing the unique hemostatic effects, even after the storage for two years at room temperature. Furthermore, the agent for promoting the healing of damaged tissue, in which a drug applicable to wounded surface is combined with the said cellulose by ionic bonding, affinity bonding, or mechanical bonding, of the present invention also has excellent storage stability.

The following Manufacturing Examples are given for the purpose of illustrating the absorbable hemostatic cellulose used in the agent for promoting the healing of wounded tissue of the present invention in further detail.

### Manufacturing Example 1: Manufacturing of soluble hemostatic cellulose fibers

35.5 g of natural or regenerated cellulose fibers were placed into a 500ml rotating reaction container at room temperature. Next, 175ml of ethanol solution, which was consisted of the 38 volumetric parts of 45% sodium hydroxide solution and of the 62 volumetric parts of 95% ethanol, was added and churned for two hours. Subsequently, in this reacted solution, monochloroacetic acid reactivity solution, which was consisted of the 40 ponderal solution of monochloroacetic acid and the 60 ponderal solution of 95% ethanol, was added and churned for six hours in the same way. In addition, the pH of the fiber obtained after completing the reaction was adjusted to 6.5-7.0 with mixture solution of 20% hydrochloric acid and 75% ethanol. Then, until the included amount of NaCl in the fiber became less than 1%, it had been washed.

The cellulose fiber, thus treated, was dried and sterilized, so that the desired absorbable hemostatic cellulose fiber was obtained. This cellulose fiber has the chemical structure unit shown in the aforementioned formula (I).

The solubility to water was examined, and it absorbed water and expanded within a few seconds. Consequently, it became colloidal state.

### Example 1: Manufacture of the agent for promoting the repairing of the damaged tissue

The absorbable cellulose, obtained in Manufacturing Example 1, was immersed in 100% ethanol solution, and to this solution was added an aqueous solution of cell growth factors in less than 25%. Then, adjusting the pH in consideration of the isoelectric point of the cell growth factors, it was churned to obtain the absorbable cellulose, which the cell growth factors were adhered to and combined with.

The combined cell growth factors are Epidermal Growth Factor (EGF), Platelet-derived Growth Factor (PDGF), Bone Morphogenetic Protein (BMP) and the like. These cell growth factors may be administered topically than systemic, and showed long acting effect. Therefore, it is possible to administer them topically over a long period of time. Furthermore, to preventing over healing, it is also possible to apply antagonism agent to cell growth factors.

### Manufacturing Example 2: Manufacturing of soluble hemostatic cellulose fibers

5.0 g of natural or regenerated cellulose fibers at room temperature was placed in a 500 ml rotating container vessel, and 350 ml of an aqueous solution of sodium hypochlorite, with a pH range of 9 to 10.5, was added, besides the mixture was churned for one hour. Then, the sodium hypochlorite was removed off, and the fibers washed with water were removed off. The fibers were again placed in a 500ml rotating reaction container, and the 350ml of 0.3% hydrogen peroxide aqueous solution and 1.4 g of sodium pyrophosphate were added. After that, the mixture was churned for one hour at 95 to 100°C at a pH range of 9 to 10.5. After the completion of the reaction, the hydrogen peroxide solution was removed, and the resulting fibers (cellulose) were washed with water.

Then, the cellulose was placed in the same 500ml-reaction container. Next, 290ml of the mixture solution of ethanol and aqueous sodium hydroxide solution, comprising of 38 volumetric parts of 45% sodium hydroxide aqueous solution and 62 volumetric parts of 95% ethanol, was added, and churned for two hours. Then, 105ml of the reactive monochloroacetic acid solution, comprising of 40 ponderal parts of monochloroacetic acid and 60 ponderal parts of 95% ethanol, was added in this reaction mixture, and churned for six hours in the same way. After the reaction, the pH of the resulting fibers was adjusted between 6.5 and 7.0 by a 20% hydrochloric acid - 75% ethanol solution, and washed with 75% ethanol until the included amount of sodium chloride in the fibers was less than 1%.

The cellulose fibers thus obtained were dried and sterilized to obtain the desired soluble hemostatic cellulose fibers. These cellulose fibers had the chemical structural unit represented by the formula (I).

The solubility of this fiber in water was examined, and it was found that water was absorbed within a few seconds and it expanded to show good colloid states.

### Example 2: Manufacture of the agent which promotes the recovery of damaged tissue

After washing the absorbable cellulose with 75% ethanol aqueous solution, the cellulose was immersed in 100% ethanol, and it was added with the weight ratio within a range of 25% after adjusting the pH level of the aqueous solution of the neovascular factors (PD-ECGF). As a result, the neovascular factors were fixed in the absorbable cellulose.

While the agent for the promotion of the repairing of damaged tissue obtained in the Examples can be applied as a healing-promotion agent to wounds, it was evident that it can also be applied to prevent reoccurrence of cancers following surgical removal of cancer tissues when anti-neovascular factors or tumor necrosis factors are fixed in the absorbable cellulose.

### INDUSTRIAL APPLICABILITY

As described above, the agent for the promotion of repairing of damaged tissue consists of a fibrous carboxymethyl cellulose, especially absorbable hemostatic cellulose, in which a drug applicable to wounded surface is bonded with the said cellulose by ionic bonding, affinity bonding or mechanical bonding.

The absorbable hemostatic cellulose, used in the present invention can be obtained through the chemical processing of natural and regenerated cellulose fibers. This absorbable hemostatic cellulose excels in the absorption of tissue fluids such as blood, and expands approximately five times as much as its original volume after absorption. Next, it dissolves rapidly when applied to the wounded surface, especially in contact with blood, and goes into a colloid state with blood raising the hemostatic effects.

Furthermore, when the agent for the promoting of the healing damaged tissue of the present invention is applied to the wounded region, it absorbs body fluids such as blood, and expands. In conjunction with the expansion of the cellulose, the drug combined with the said cellulose is released to heal the damaged tissue effectively. Therefore, it can be used clinically, too, and the effect is excellent.

## Claims

1. An agent for repairing the damaged tissue consisting of a fibrous carboxymethyl cellulose, in which a drug applicable to wounded surface is combined with the said fibrous carboxymethyl cellulose by ionic bonding, affinity bonding or mechanical bonding.

2. The agent for repairing the damaged tissue as claimed in Claim 1, wherein the fibrous carboxymethyl cellulose is fibrous soluble hemostatic cellulose.

3. The agent for repairing the damaged tissue as claimed in Claim 2, wherein the fibrous absorbable hemostatic cellulose has a good absorbency of body fluid, that is obtained from natural or regenerated cellulose by processing with an alcoholic aqueous solution of alkali metal hydroxide, and reacting with monohalogenated acetic acid to introduce the carboxymethyl group into the hydroxyl group presented in the anhydrous glucose unit of the cellulose molecules, and then purifying.

4. The agent for repairing the damaged tissue as claimed in Claim 2, wherein the fibrous absorbable hemostatic cellulose has a good absorbency of body fluid, that is obtained from natural or regenerated cellulose by processing with an alcoholic aqueous solution of alkali metal hydroxide, and reacting with monobalogenated acetic acid to introduce the carboxymethyl group into the primary hydroxyl group presented in the anhydrous glucose unit of the cellulose molecules, and then purifying.

5. The agent for repairing the damaged tissue as claimed in Claim 3 or 4, wherein the fiber soluble hemostatic cellulose is obtained from natural or regenerated cellulose by processing with an aqueous solution of sodium hypochlorite and hydrogen peroxide, and followed by carboxymethylization and purification.

6. The agent for repairing the damaged tissue as claimed in Claim 1, wherein the drug that can be applied to a wounded surface is at least one selected from the group of cell growth factors, cell growth factor antagonism, antibacterial, agents, antifungal agents, disinfectant, steroids, anti-inflammatory agents and antihistamines.

7. The agent for repairing the damaged tissue comprising of any one of the soluble hemostatic cellulose as claimed in Claim 2 to 5, in which the drug that can be applied to at least one wounded surface, selected from the group of cell growth factors, cell growth factor antagonism, antibacterial agents, antifungal agents, disinfects, steroids, anti-inflammatory agents and antihistamines, is combined with the said fibrous carboxymethyl cellulose by ionic bonding, affinity bonding or mechanical bonding.

8. A soluble hemostatic cellulose as claimed any one of claims 2 to 5 used by the agent for repairing the damaged tissue as claimed in Claim 1.
